# EUROPEAN PATENT APPLICATION

(11) **EP 1 293 212 A1**
(43) Date of publication of application: **19.03.2003**
(21) Application number: 02255834.0
(22) Date of filing: 21.08.2002
(51) Int. Cl.: A61K 39/00, A61P 35/00

(54) **Anti cancer vaccine comprising whole cancer cells with modified sialic acid**

(30) Priority: 21.08.2001 US 313466 P
(71) Applicant: NATIONAL RESEARCH COUNCIL OF CANADA, Ottawa Ontario K1A OR6 (CA)
(72) Inventor: Jennings, Harold J., Gloucester, Ontario K1J 6G6 (CA); Liu, Tianmin, Ottawa, Ontario K1R 7B5 (CA); Yang, Qingling, Gloucester, Ontario K1W 1J2 (CA)
(74) Representative: McKechnie, Neil

(57) **Abstract**

When tumor cells are incubated with N-propionyl mannosamine, the N-acetyl groups of their surface a2-8 polysialic acid are converted to N-propionyl groups. The resultant bio-engineered cancer cells can be killed and used as a allogenic or autologous therapy or vaccine. The presence of N-propionylated polysialic acid-specific antibodies is detected in animals immunized with the vaccine prior to tumor implantation. Mice immunized with the heat-killed cancer cells experience better protection against challenge with live autologous RMA-S cells than mice immunized with heat-killed autologous RMA-S cells. Killed cells having modified sialic acid groups on their surface may be used as an anti-cancer therapy or vaccine either alone or in combination with an anti-cancer compound, such as cyclophosphamide.

## Description

This application claims the benefit of priority under 35 USC 119 (e) of U.S. Provisional Patent Application No. 60/313,466 filed August 21, 2001.

### FIELD OF THE INVENTION

The present invention relates generally to cancer prevention and anti-cancer therapy.

### BACKGROUND OF THE INVENTION

The permissiveness of the enzymes involved in sialic acid biosynthesis and sialoside formation has been explored for the bioengineering of cell surface molecules (1-10). This strategy was first reported by Reutter and co-workers (2) who demonstrated that exposure of mammalian cells in tissue culture and in vivo to different N-acylmannosamine precursors resulted in the expression of unnatural N-acylated sialic acid residues on the cell surface glycoconjugates. Bertozzi and co-workers (6,7) have exploited this enzymatic permissiveness further by successfully using N-levulinoylmannosamine as the precursor to introduce N-levulinoylsialic acid residues on the surface of a number of human cell lines. This procedure has therapeutic potential because it introduces unique active keto groups on the surface of the cells which via the use of appropriate chemical reagents, can be used for the chemotargeting of drugs.

We and others have also reported (9,10) the successful application of enzymatic permissiveness in the biosynthesis of sialic acid to the immunotherapy of cancer cells which could further the development of efficacious carbohydrate-based vaccines. Although some success has been reported (11) in creating cancer vaccines based on synthetic cell surface glycoconjugate vaccines, the area remains problematic, due to the fact that cancer cells fail to produce markers that distinguish sufficiently from normal cells. We demonstrated (9) that when mouse and rat leukemic cells were incubated with N-propionylmannosamine (ManNPr) their surface a2-8 polysialic acid was converted to N-propionyl polysialic acid (NPr polysialic acid). Expression of this unnatural antigen on the surface of the tumor cells induced their susceptibility to cell death mediated by NPr polysialic acid-specific antibody. Furthermore, this antibody was also able to effectively control metastasis in a solid tumor model, when mice were administered the precursor ManNPr. Lemieux and Bertozzi (10) were also able to demonstrate a similar *in vitro* cytotoxic effect on cancer cells, previously incubated with N-levulinoylmannosamine; in the presence of antibody raised using an N-levulinoylsialic acid-KLH conjugate.

In International Publication No. WO 01/09298, Jennings *et al.* disclose a mammalian cancer cell having a cell surface marker incorporating modified sialic acid units capable of initiating an immune response in a mammalian system containing them which is sufficiently strong to effectively combat proliferation of such cells. The modified sialic acid marker can be produced by providing mammalian cancer cells with a chemically modified precursor of such a sialic acid unit, for example, an N-acylated precursor such as an N-acylated mannosamine. This document teaches the use of antibodies produced by these cells, but does not teach use the cell itself as a therapy or vaccine.

### SUMMARY OF THE INVENTION

According to the invention, we now report another application of the enzymatic permissiveness associated with sialic acid biosynthesis for the preparation of modified whole cell cancer vaccines based on both heat-killed N-propionylated autologous and allogeneic cancer cells.

The invention provides a killed immunogenic mammalian cancer cell having a cell surface marker incorporating a modified sialic acid unit capable of initiating an immune response in a mammalian system containing them which immune response is sufficiently strong to effectively combat proliferation of a live cancer cell.

The invention further provides an anti-cancer treatment comprising a pharmaceutically effective amount of a killed immunogenic mammalian cancer cell having a cell surface marker incorporating a modified sialic acid unit capable of initiating an immune response in a mammalian system containing them which immune response is sufficiently strong to effectively combat proliferation of a live cancer cell, in combination with a pharmaceutically acceptable carrier.

Further, the invention provides an anti-cancer treatment comprising a pharmaceutically effective amount of a killed immunogenic mammalian cancer cell having a modified sialic acid unit on the surface thereof, said modified sialic acid unit comprising a N-propionyl polysialic acid, said cell being formed by, prior to killing the cell, incubating the cell with N-propionyl mannosamine, and subsequently killing the cell, and combining the cell with a pharmaceutically acceptable carrier.

Other aspects and features of the present invention will become apparent to those ordinarily skilled in the art upon review of the following description of specific embodiments of the invention in conjunction with the accompanying figures.

### BRIEF DESCRIPTION OF THE DRAWINGS

Embodiments of the present invention will now be described, by way of example only, with reference to the attached Figures.

**Figure 1A** illustrates NPr polysialic acid expression on the surface of tumor cells for RMA-S cells incubated with different concentrations of ManNPr in cell culture for 1 day.

**Figure 1B** illustrates NPr polysialic acid expression on the surface of tumor cells for P815 cells incubated with 1 mg/ml ManNPr in cell culture.

**Figure 2** shows that heating-killing of live NPr RMA-S tumor cells did not greatly effect cell integrity, as determined by similar patterns obtained through flow cytometry.

**Figure 3** illustrates similar expression of NPr-polysialic acid on live and heat-killed cells, as measured by flow cytometry using MAb 13D9.

**Figure 4** shows production of NPr-polysialic-specific antibodies in mice immunized with heat-killed NPr RMA-S tumor cells, versus mice injected with RMA-S and normal mice.

**Figure 5A** illustrates the specificity of antibodies for NPr-polysialic acid in mice immunized with NPr RMA-S tumor cells.

**Figure 5B** illustrates the specificity of antibodies for polysialic acid from mice immunized with NPr RMA-S tumor cells.

**Figure 6A** shows protection of mice from tumor growth by vaccination with heat-killed autologous and NPr RMA-S tumor cells in a control group.

**Figure 6B** shows protection of mice from tumor growth by vaccination with heat-killed autologous and NPr RMA-S tumor cells in an autologous RMA-S vaccine group.

**Figure 6C** shows protection of mice from tumor growth by vaccination with heat-killed autologous and NPr RMA-S tumor cells in NPr RMA-S vaccine group.

**Figure 7** illustrates NPr-polysialic acid expressed on the surface of RMA-S cells with and without pretreatment with 2.5 mg/ml ManNPr for 48 hour at 37 °C.

**Figure 8** shows that antibody titer in sera from the group treated by vaccine and cyclophosphamide (CY) versus cyclophosphamide alone, and normal mice.

**Figure 9** illustrates sera polysialic acid binding for groups treated by vaccine and cyclophosphamide, versus cyclophosphamide alone and normal mice .

**Figure 10** provides a comparison of antibodies from the group treated by vaccine and cyclophophaminde against both NPr- and NAc- polysialic acids.

**Figure 11** illustrates the effect of vaccine on the growth of tumor *in vivo.*

### DETAILED DESCRIPTION

Generally, the present invention provides a killed cell useful in anti- cancer therapy and prevention.

Abbreviations used herein include: ManNPr, N-propionyl-D-mannose; NPr polysialic acid, N-propionylated polysialic acid; FBS, fetal bovine serum; PBS, phosphate-buffered saline; PBST, phosphate-buffered saline containing Tween 20.

The invention provides a killed immunogenic mammalian cancer cell having a cell surface marker incorporating a modified sialic acid unit capable of initiating an immune response in a mammalian system containing them which immune response is sufficiently strong to effectively combat proliferation of a live cancer cell. Optionally, the modified sialic acid unit comprises an N-propionyl group, for example, as derived from N-propionyl mannosamine.

The killed immunogenic mammalian cancer cell may be either autologous to or allogeneic to the mammalian system. The cell may be killed by any acceptable method, such as for example, heat-killing.

The invention further provides an anti-cancer treatment comprising a pharmaceutically effective amount of a killed immunogenic mammalian cancer cell having a cell surface marker incorporating a modified sialic acid unit capable of initiating an immune response in a mammalian system containing them which immune response is sufficiently strong to effectively combat proliferation of a live cancer cell, in combination with a pharmaceutically acceptable carrier.

The anti-cancer treatment may comprise co-administration of the killed cell with N-propionyl mannosamine. Further, the treatment may comprise co-administration of the killed cell with an anti-cancer drug selected from the group consisting of cyclophosphamide, melphalan, adriamycin, decarbazine, armustine, cisplatin, tamoxifen, bleomycine, vincristine and lomustine.

The invention further provides the use of a killed immunogenic mammalian cancer cell, as described herein, for preparation of an anti-cancer medicament.

Cells may be killed for use in the invention according to any feasible method, for example, the cells can be killed by heat or radiation. Any manner of killing cells as is known in the art may be used.

The killed cell according to the invention is used as an antigen, and is capable of raising high levels of antibody. As a result the tumour burden is reduced, and growth may be stopped. Killing or regression of the tumours may be conducted in a separate step, after administration of the vaccine according to the invention.

According to the instant invention, the killed cell may be used alone or in combination with N-propionyl mannosamine, which is herein referred to interchangeably as "the precursor" or "the precursor compound". It is not necessary to use the precursor compound, but it may be useful in enhancing the response of the individual. The invention illustrates that reduction and/or elimination of metastasis without use of the precursor is possible simply using the killed cell according to the invention. With N-propionyl groups on the cell surface, metastasis is stopped/prevented. By "N-propionyl groups" it is meant any group derived from N-propionyl mannosamine. The use of the precursor alone does not prevent metastasis as effectively as does use of the cell alone does. However, N-propionyl mannosine has been used against meningitis so it is known to be immunogenic.

Autologous tumours may be used (from the same individual), and allogenic tumours (same species different individual).

The mice used in the experimental data provided herein weigh from about 40 g to about 60g so a putative dose for a human can easily be calculated on a body weight basis by one of skill in the art, with consideration given to the transferrability of dose amounts on a body-weight basis between mice and humans.

The examples herein illustrate the use of the killed cells according to the invention in combination with a well-known anti-cancer (immunosupressive) drug: cyclophosphamide. Other drugs which may be used in combination with the killed cells of the instant invention (as a vaccine or therapy) include such drugs as Melphalan, Adriamycin, Decarbazine, carmustine, cisplatin, tamoxifen, bleomycine, vincristine and lomustine, but examples are not limited to these.

Because the inventive protocol does not require the use the precursor, it is distinct from prior art teaching, for example WO 01/09298 in which the precursor had to be used to get rid of the tumour. Further, in WO 01/09298 the mice were immunized before treatment to give them tumours (a preventative regime). According to the instant invention, the use of the killed cell is therapeutic, as the cell is given after the animal has tumours and the precursor can then be used as well. Thus, in the invention, the precursor may be used after boosting the immune system, but is not required to boost it initially.

In an alternative embodiment, antibodies to the cell can be acquired, and then the precursor is used to render endemic cancers immunogenic and open to killing by the animal's own immune system.

According to the invention, when tumor cells (RMA-S) are incubated with N-propionyl mannosamine, the N-acetyl groups of their surface a2-8 polysialic acid are converted to N-propionyl groups. The resultant bio-engineered cancer cells are then killed and used as a vaccine. The presence of N-propionylated polysialic acid-specific antibodies is detected in mice immunized with the vaccine, and in addition, mice immunized with the vaccine experience better protection against challenge with live autologous RMA-S cells than mice immunized with killed autologous RMA-S cells (non-engineered cells). Significant protection is also obtained in mice challenged with RMA-S using a vaccine comprising killed and similarly bio-engineered allogeneic mouse tumor cells (P815). Like RMA-S, these cells also exhibit strong binding to MAb 13D9 when incubated with N-propionyl-mannosamine but in this case only the previously bio-engineered P815 cells afforded protection.

### EXAMPLE 1

### EXPERIMENTAL PROCEDURES

***Cell Lines.*** Mutant mouse lymphoma (RMA-S) obtained from the original RMA cell line was derived from C57BL/6 mice (12). P815 mastocytoma from mouse strain DBA/2 as obtained from the American Type Culture Collection (Manassas, VA). All cells were cultured in RMPI1640 medium with 8% FBS.

***Mice.*** Female C57BL/6 mice were purchased from Charles River (Quebec, Canada) and maintained in our animal facility.

***Monoclonal Antibodies .*** MAb 13D9, specific for NPr polysialic acid has been previously described (13); MAb735 specific for polysialic acid (14), was the gift of Prof. D. Bitter-Suermann (Medizinische Hochschule, Hannover, Germany).

***ELISA.*** Total IgG antibody was measured by ELISA. HSA conjugates of NAc- and NPr-polysialic acids (11KDa fractions), synthesized from Colominic acid (Nacalai Tesque, Kyoto, Japan) as previously described (13), were used as coating antigens. The wells of flexible PVC microtiter plates (Falcon, NJ, USA) were coated with 50 µl of a solution of the HSA conjugates (0.5 µg/ml PBS). The plates were then washed (three times) with PBS and Tween-20 (0.05%) (Allen Fisher Assoc., Haddonfield, NJ), followed by blocking with 150 µl of 10% FBS in PBS for 1 hr at room temperature. The contents of the wells were removed and serial dilutions (50 µl/well) of antibody in PBS buffer containing 0.05% Tween-20 (PBST) were performed, followed by incubation for 60 min at ambient temperature. The wells were then reacted, after washing three times in PBST, with 50 µl of streptavidin-horseradish peroxide anti-mouse IgG conjugate (1 µg/ml) (Kirkegaard & Perry Laboratories, Gaitersburg, MD) in 10% BSA/PBST and incubated for 1h at room temperature. The wells were washed five times with PBST and 100 µl of substrate 2,2'-azino-bis (3-ethylbenzthiazoline-6-sulfonic acid) (1 mg/ml) in 44 mM Na₂HPO₄, 20 mM citric acid and 0.3% H₂O₂. The plates were read at 405 nm with a reference wavelength of 490 nm.

***Vaccine Preparation and Administration.*** Cancer cells were incubated with ManNPr (1 mg/ml) for 48h, and after washing with PBS, they were suspended in PBS and killed by heating at 70° for 10 min. Mice were immunized with 2 x 10⁶ cells and boosted with the same amount of vaccine after a month. 10 days after boosting the mice were injected subcutaneously with 1 x 10⁶ live RMA-S tumor cells in the shaven area of the rear flank. Tumor growth was monitored routinely.

***Flow Cytometry.*** For flow cytometry, cells were incubated with MAbs 13D9 or 735 in 50 µl of RPMI + 1% FBS on ice for 30 min. The cells were then washed and incubated with FITC-labelled secondary antibodies. After 30 min the cells were washed and fixed in 1% formaldehyde and assayed on a flow cytometer (Coulter Incorporation, Miami, FL).

### RESULTS

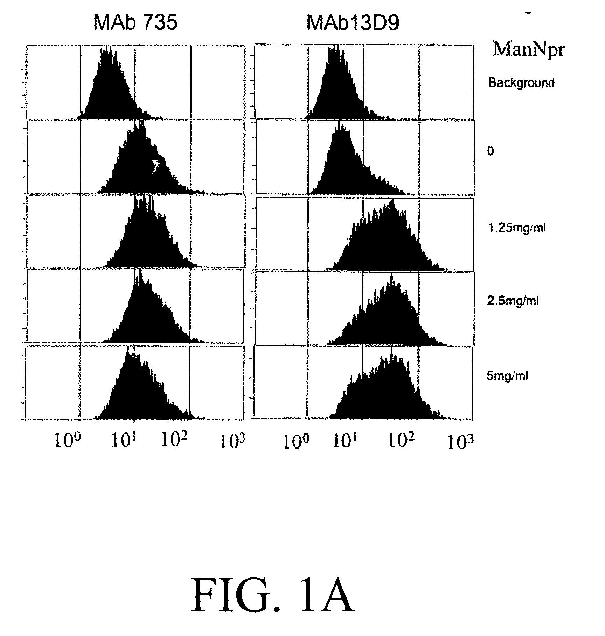

***Preparation of vaccines.*** RMA-S cells derived from leukemic cell line RMA, are defective in transportation-associated proteins (12). However, RMA-S cells like those of the original RMA cells (9), can still incorporate ManNPr into their surface polysialic acid as shown by flow cytometric analysis. **Figure 1A** illustrates NPr polysialic acid expression on the surface of tumor cells for RMA-S cells incubated with different concentrations of ManNPr in cell culture for 1 day. Following harvesting of the cells the expression of polysialic acid and its NPr analog were measured by flow cytometry using MAb 735 and MAb 13D9 respectively.

When treated with ManNPr at different concentrations for 48 h, the surface polysialic acid was significantly converted to NPr polysialic acid, as determined by staining with an Ab13D9, specific for NPr polysialic acid (13). In addition the conversion was slightly increased using higher concentrations of ManNPr. RMA-S cells were also stained with MAb 735 specific for polysialic acid (14), and binding to the cell surface became weaker with exposure of the cells to increasing amounts of ManNPr. Heat-killed RMA-S cells pretreated with 1 mg/ml ManNPr for 48 h, were then used as the autologous vaccine.

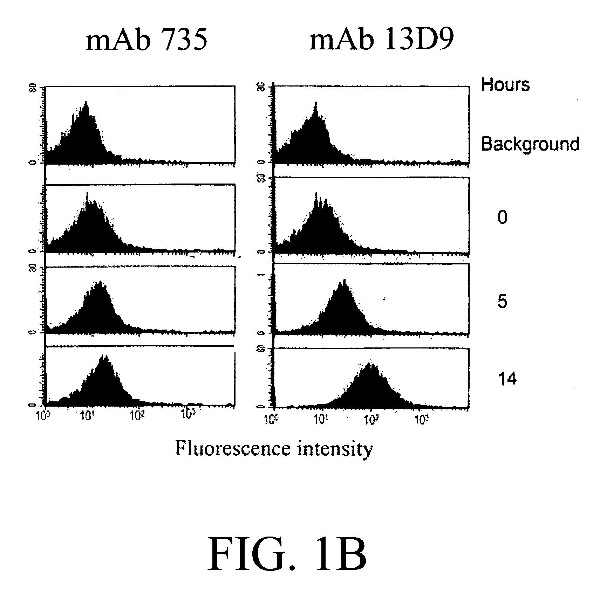

To identify a cell line allogeneic to RMA-S, but which was also able to express polysialic acid, a number of allogeneic tumor cell lines were screened by flow cytometric analysis using MAb 735 (data not shown). Only one cell line P815, which was derived from DBA/2 mice, exhibited a fluorescence shift, albeit of small magnitude. **Figure 1B** illustrates NPr polysialic acid expression on the surface of tumor cells for P815 cells incubated with 1 mg/ml ManNPr in cell culture. The cells were harvested at intervals and the expression of polysialic acid and its NPr analog were measured as described above. P815 cells were treated with ManNPr for different time periods, and after washing, they were stained with either MAb 735 or MAb 13D9. Flow cytometric analysis (**Figure 1B**) showed that there was a large shift of fluorescence on P815 cells when they were stained with MAb 13D9, and in addition there was even a slight increase in the binding of MAb 735 to the surface of P815 cells following incorporation of ManNPr. Obviously the transformation of polysialic acid on the surface of P815 cells follows a different pattern from RMA-S RMA and RBL-2H3 (9) cell lines that already have higher initial levels of expression of polysialic acid. Heat-killed P815 cells, pretreated with ManNPr, were then used as the allogeneic vaccine.

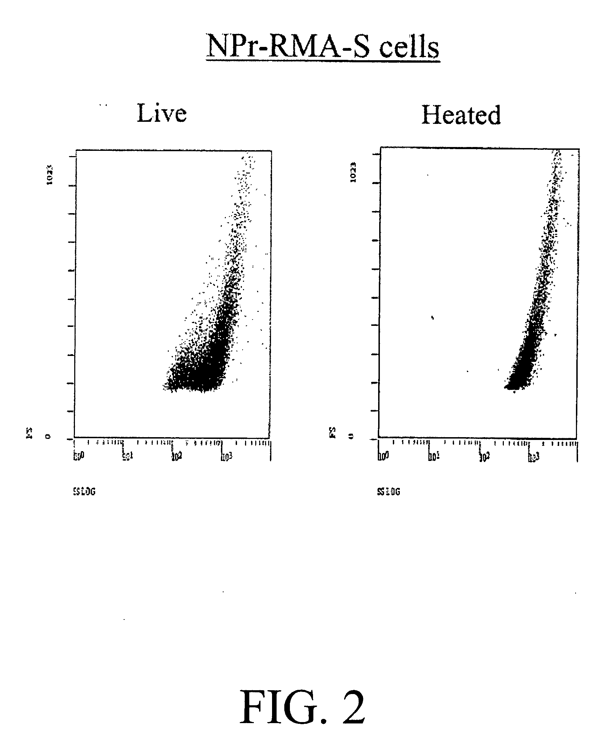

While heating is a practical way to kill tumor cells, it was important to confirm that this treatment did not interfere with the structural integrity of the dead cells prior to their use as vaccines. Firstly, it was ascertained by flow cytometric analysis that RMA-S cells, that had been previously incubated with ManNPr before heat-killing, maintained a similar pattern to the live cells as depicted in the diagram of forward and side scatters. **Figure 2** shows that heating-killing of live NPr RMA-S tumor cells did not greatly effect cell integrity, as determined by similar patterns obtained through flow cytometry.

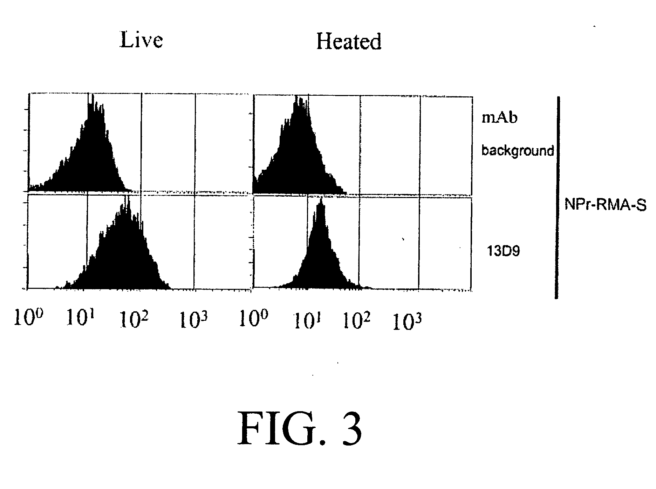

Secondly, it was also demonstrated by flow cytometric analysis, using MAb 13D9 that RMA-S cells, previously incubated with ManNPr and then subsequently heat-killed, still maintained their surface NPr polysialic acid. Figure 3 illustrates similar expression of NPr-polysialic acid on live and heat-killed cells, as measured by flow cytometry using MAb 13D9.

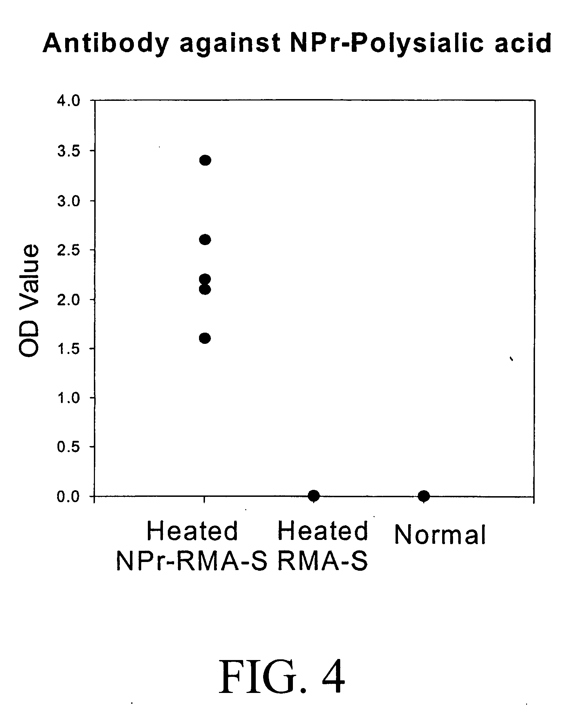

***Induction of NPr polysialic acid-specific antibodies.*** Heat killed RMA-S cells, pretreated with ManNPr, were injected intraperitoneally in mice. After one month the mice were boosted using the same heat-killed cells and seven days later were bled by the tail. Total IgG NPr polysialic acid-specific antibody was measured by ELISA using an NPr polysialic acid-HSA conjugate as the coating antigen. **Figure 4** shows production of NPr-polysialic-specific antibodies in mice immunized with heat-killed NPr RMA-S tumor cells, versus mice injected with RMA-S and normal mice. Significant titers of NPr polysialic acid-specific IgG antibody were induced in all the mice immunized with the NPr RMA-S vaccine, whereas the antisera induced in the unimmunized mice and those immunized with the RMA-S heat-killed vaccine contained no antibodies that bound to NPr polysialic acid. Total IgM NPr polysialic acid-specific antibody was also measured in the above antisera, using the same procedure, and the results were similar to those depicted in Figure 4 (data not shown).

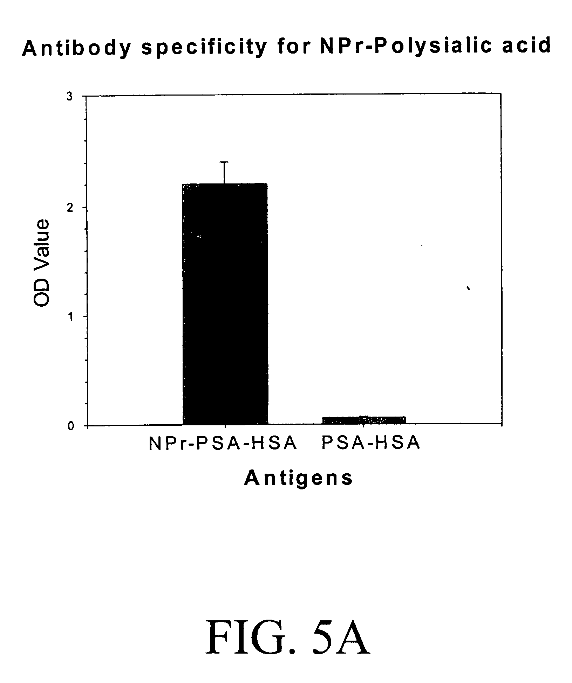

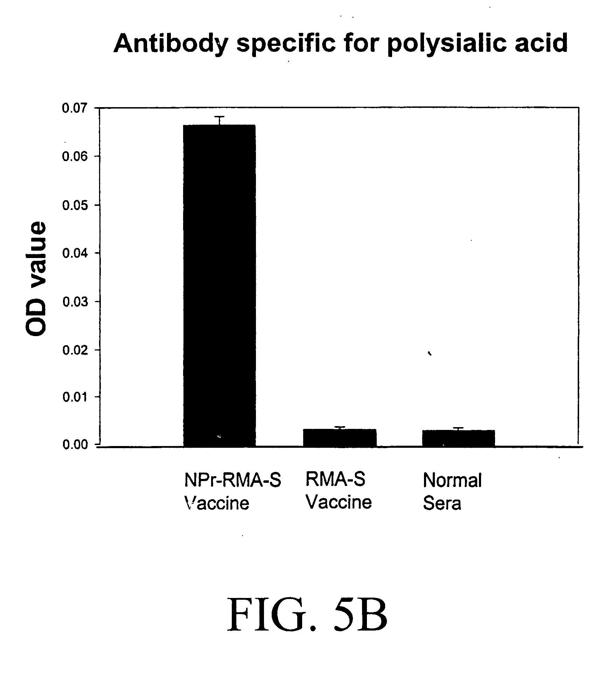

The specificity of the IgG NPr polysialic acid antibodies induced in mice by the heat-killed NPr RMA-S whole cell vaccine was determined by ELISA using both polysialic acid- and NPr polysialic acid-HSA conjugates as coating antigens. The results are shown in **Figure 5A,** and indicate that while antibody from mice immunized with NPr RMA-S vaccine bind strongly to NPr polysialic acid, only relatively weak binding to polysialic acid was observed. That this antibody binding to polysialic acid was mainly due to the induction of cross-reactive antibody by the NPr RMA-S vaccine was supported by the fact that both a control antiserum and that induced by the heat-killed RMA-S vaccine bound even less strongly to polysialic acid (**Figure 5B**).

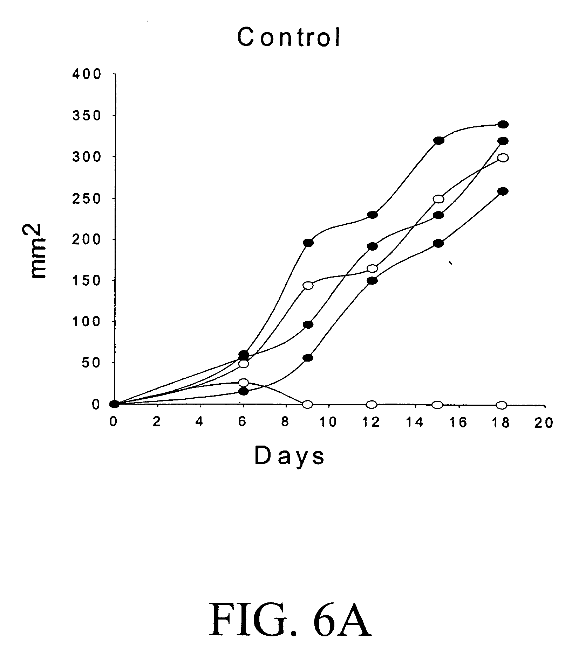

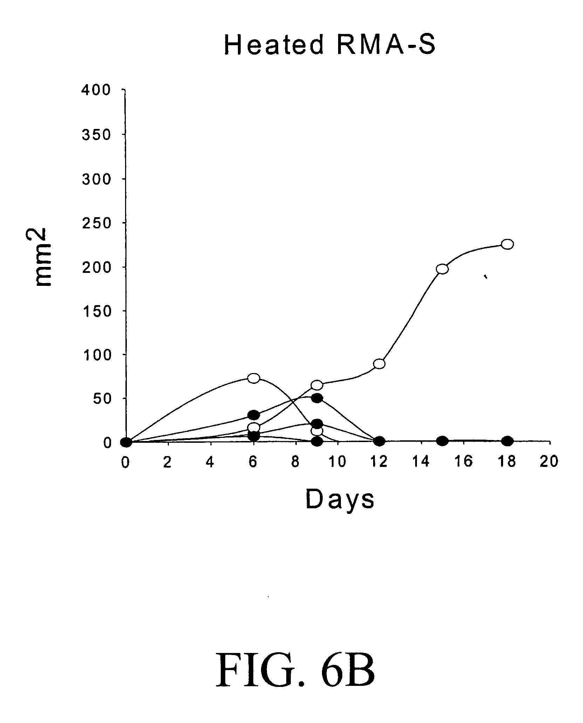

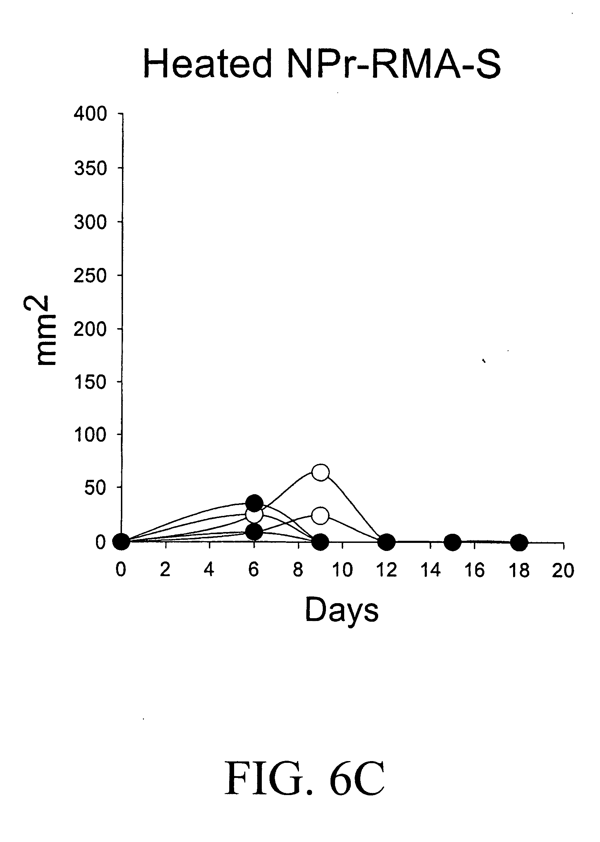

***Effect of N-propionylated cancer cell vaccines on tumor growth.*** To determine whether the above vaccines could control tumor growth, a mouse solid tumor model was established. Mice were immunized with heat-killed RMA-S cells or their bio-engineered N-propionylated analog NPr RMA-S (2 x 10⁶ cells per mouse), and boosted with the same vaccine one month later. After ten days the above mice plus an unvaccinated control group were inoculated subcutaneously in the rear flank with live autologous RMA-S tumor cells (1 x 10⁶ cells per mouse). Tumor size was monitored routinely using calipers and the results of the experiments are shown in **Figures 6A** to **6C.** In the unvaccinated group four of the five mice developed tumors (**Figure 6A**), whereas considerable protection was afforded in both vaccinated groups. Only one of the mice in the group given the heat-killed RMA-S cell vaccine developed a tumor (**Figure 6B**), and none was observed in the mice immunized with the analogous NPr RMA-S cell vaccine (**Figure 6C**).

To provide statistical significance to this result we repeated the above experiment using more mice. The results are shown in **Table 1** and confirm that the heat-killed NPr RMA-S vaccine provided significantly better protection against challenge by live autologous RMA-S cells (15% developed tumors) than the equivalent RMA-S vaccine (50% developed tumors). In the unvaccinated group 80% of the mice developed tumors. Because of the high degree of protection provided by the unpropionylated autologous RMA-S vaccine in the above experiment, a similar but potentially more clear-cut experiment was carried out using an allogeneic polysialylated cancer cell line (P815) to prepare the vaccines. Using this strategy we hoped to better delineate the contribution to protection provided by N-propionylation.

**Table 1**

| Protection of mice from tumor acquisition by heat-killed autologous vaccine of NPr RMA-S cells | | | |
|---|---|---|---|
| | Control | Heat-Killed RMA-S | Heat-Killed N-propionyl RMA-S |
| Tumor | 16/20 | 5/10 | 3/20 |
| Percentage | 80% | 50% | 15% |
| Mice were immunized with heat-killed RMA-S or NPr RMA-S cells. After boosting with same vaccine, mice were subcutaneously inoculated with 1 X 10⁶ RMA-S cells at day 10. Tumors were routinely monitored following their inoculation. | | | |

The vaccination and challenge protocols followed were identical to those used beforehand with the RMA-S cell vaccines, and the results are shown in **Table 2.** The heat-killed NPr P815 vaccine gave significantly more protection in terms of tumor acquisition (58% of the mice developed tumors) than the unpropionylated analog (80% of the mice developed tumors), but much less protection than that given by the autologous NPr RMA-S vaccine (only 10% of the mice developed tumors). Significantly in this latter experiment the unpropionylated P815 vaccine gave no protection at all against tumor establishment when compared to the control groups (70% of the mice developed tumors).

**Table 2**

| Protection of mice from tumor acquisition by allegoric vaccine of NPr P815 cells | | | | |
|---|---|---|---|---|
| | Control | Heat-Killed NPr RMA-S | Heat-Killed P815 | Heat-Killed NPr P815 |
| Tumor | 7/10 | 1/10 | 8/10 | 7/12 |
| Percentage | 70% | 10% | 80% | 58% |
| Mice were immunized with heat-killed NPr RMA-S cells, P815, or NPr P815 cells. After boosting with same vaccine, mice were subcutaneously inoculated with 1 X 10⁶ RMA-S cells at day 10. Tumors were routinely monitored following their inoculation | | | | |

***DISCUSSION***

a2-8 Polysialic acid is found on a number of important cancers (15,16,17) and there is strong evidence that it is associated with metastasis (16,18). In previous studies we had demonstrated that bioengineering of the surface a2-8 polysialic acid of rat mouse leukemic tumor cell lines enhances their susceptibility to antibody mediated cytotoxicity (9). Incubation of the tumor cells with ManNPr resulted in the substitution of the N-acetyl groups of the surface polysialic acid by N-propionyl groups. Expression of the altered surface a 2-8 polysialic acid induced the antigen-specific in vitro cytotoxicity of the cancer cells mediated by a 2-8 NPr polysialic acid-specific monoclonal antibody. Facile production of this antibody was accomplished using a highly immunogenic a 2-8 NPr polysialic-protein conjugate vaccine (13). Of particular importance we were also able to demonstrate, in an in vivo mouse solid tumor model, that a2-8 NPr polysialic acid-specific antibody was able to effectively control metastasis, when mice were administered the precursor ManNPr prior to being challenged with live RMA tumor cells (9).

Recently in similar in vitro studies it was demonstrated (10) that altering the surface sialic acid of cancer cells by using N-levulinoylmannosamine as precursor, also induced their cytotoxicity to antibodies. These polyclonal antibodies were raised using a multivalent N-levulinoylsialic acid-KLH conjugate vaccine.

We now report another application of the above technology in which heat-killed bioengineered autologous cancer cells are used as vaccines themselves. Killed whole autologous cancer cells have been tried as human cancer vaccines, albeit with only limited success (19,20). This is probably due to a number of reasons, not the least of which is that tumor antigens are weakly immunogenic and often induce tolerance rather than effective immunity. However, a novel approach to active cancer immunotherapy, based on the chemical modification of autologous tumor cells, has been reported (20). This approach involves the introduction of the hapten dinitrophenyl (DNP) into the surface protein antigens of autologous cancer cells. This enhances the immunogenicity of the hapten substituted proteins to the extent that therapeutic cancer vaccines based on this technology, have had some success in human clinical studies.

Our strategy involves the bioengineering of the surface sialylated carbohydrate antigens of autologous tumor cells, prior to their use as vaccines. RMA-S cells when incubated with ManNPr were shown to express NPr polysialic acid on their surfaces. When used as a preventative vaccine in a mouse solid tumor model the heat-killed N-propionylated RMA-S vaccine gave increased protection against tumor development when compared to that provided by the analogous unmodified autologous RMA-S vaccine (**Table 1**). We have some evidence to suggest that this protection is mediated by an immune response to the NPr polysialic acid on the surface of the NPr RMA-S vaccine but until more evidence is forthcoming we cannot dismiss the possibility that other secondary effects, generated by the presence of NPr polysialic acid on the surface of the cells, contribute to this protection.

In addition the possibility that other N-propionylated surface carbohydrate antigens might also be involved in this protection cannot be excluded.

Additional evidence that NPr polysialic acid is involved directly in immune protection was provided by carrying out an experiment in which the surface polysialic acid of an allogeneic cancer cell line (P815) was N-propionylated by incubation with ManNPr. When used as a vaccine in mice the heat-killed allogeneic NPr P815 cells still afforded some protection against challenge by live RMA-S tumor cells, but less than that provided by the autologous heat-killed NPr RMA-S vaccine as shown in **Table 2**.

When murine tumor cells (RMA-S) are incubated with N-propionyl mannosamine, the N-acetyl groups of their surface a2-8 polysialic acid are converted to N-propionyl groups, as determined by flow cytometric analysis using an a 2-8-polysialic acid-specific monoclonal antibody (MAb 13D9). The resultant bio-engineered cancer cells are then heat-killed and used as a vaccine in mice together with a control vaccine consisting of the original heat-killed autologous tumor cells. The presence of N-propionylated polysialic acid-specific antibodies is detected only in mice immunized with the former, and in addition, mice immunized with the heat-killed bio-engineered cancer cells vaccine experience better protection against challenge with live autologous RMA-S cells than mice immunized with heat-killed autologous RMA-S cells. Interestingly, reduced but still significant protection is also obtained in mice challenged with RMA-S using a vaccine comprising heat-killed and similarly bio-engineered allogeneic mouse tumor cells (P815). Like RMA-S, these cells also exhibit strong binding to MAb 13D9 when incubated with N-propionyl-mannosamine but in this case only the previously bio-engineered P815 cells afforded protection.

### EXAMPLE 2

### EXPERIMENTAL PROCEDURES

***Cell lines.*** Mutant mouse lymphoma (RMA-S) was obtained from the original cell line derived from C57BL/6 mice. Cells were cultured in RPMI1640 medium with 8%FBS.

***Mice.*** Female C57BL/6 mice were purchased from Charles Rivers (Quebec, Canada)

***Materials.*** Polysialic acids-NAc and NPr polysialic acids (11-kDa fractions) were obtained from colominic acid as previously described. Monoclonal antibodies-mAb 13D9 is a specific antibody against NPr polysialic acid and mAb735 is a specific antibody against NAc polysialic acid. Sera were collected by the tail bleeding of mice and kept in freezer before it was used.

***ELISA.*** Total IgG antibody was measured by ELISA. Each well of ELISA plate was coated with HAS conjugates (0.5 µl /50 µl PBS/well) of NAc or NPr polysialic acids (11 kDa fractions), and blocked with 150 µl of 10%FBS in PBS. The plates were then washed three times with PBS and Tween20 (0.05%). Sera were added into well of plate after serial dilutions. The plates were kept for 1 hour at room temperature. After washing, anti-mouse IgG conjugates with horseradish peroxide (50 µl) were added into each well of plate and incubated for 1 hour at room temperature. Plates were further washed five times with PBS and Tween20 (0.05%) and 100 µl of substrate 2,2'-azino-bis-(3-ethylbenthiazoline-6-sulfonic acid)(1mg/ml) in 44mM Na₂HPO₄, 20 mM citric acid and 0.3% H₂O₂. The plates were read at 405 nm with reference wavelength of 490 nm.

***Flow cytometry.*** Cells were incubated with mAbs 13D9 or 735 in 50 µl of RPMI1640 +1% FBS on ice for 30min. The cells were then washed and incubated with FITC-labelled secondary antibodies. After 30 min the cells were washed and fixed in 1% formaldehyde and assayed on a flow cytometer.

***Vaccine preparation and administration.*** RMA-S cells were incubated with ManNPr (1mg/ml) for 48 hour, and after washing with PBS, they were suspended in PBS and killed by heating at 70 °C for 10 min. Mice were injected subcutaneously with 2 x 10⁶ RMA-S cells at the area of rear flank to develop a solid tumor. Mice were pretreated with cyclophosphamide (4 mg/mouse) at day 7 after grafting tumor. Whole cell vaccines (2 x 10⁶ cells) were inoculated at day 8 and tumor growth was monitored routinely.

Limiting dilution-Cell suspension of the mouse spleen was prepared in RPMI 1640 medium. One fifth of each spleen suspension was used to initiate a 2-fold serial dilution with complete RPMI 1640 medium and the cells were cultured over 20 days. Cancer cells of metastasis of spleen were examined under the microscope.

### RESULTS

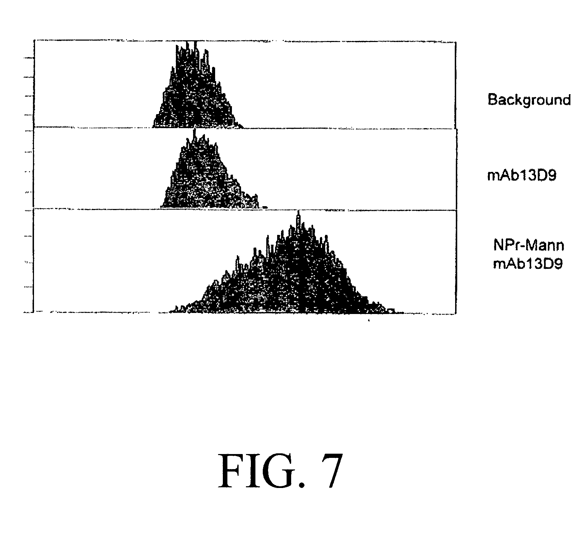

**Figure 7** illustrates Npr-polysialic acid expressed on the surface of RMA-S cells with and without pretreatment with 2.5 mg/ml ManNPr for 48 hour at 37 °C.

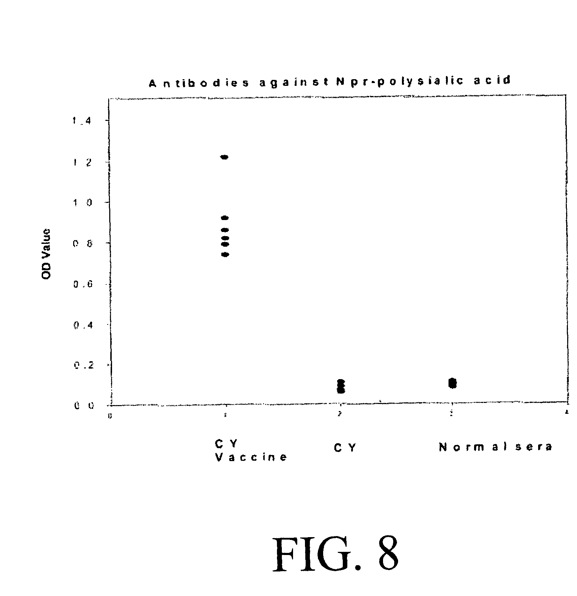

**Figure 8** shows that sera from the group treated by vaccine and cyclophosphamide (CY) have a higher antibody titer against NPr polysialic acid than the sera from the group treated with cyclophosphamide alone, and the normal mice.

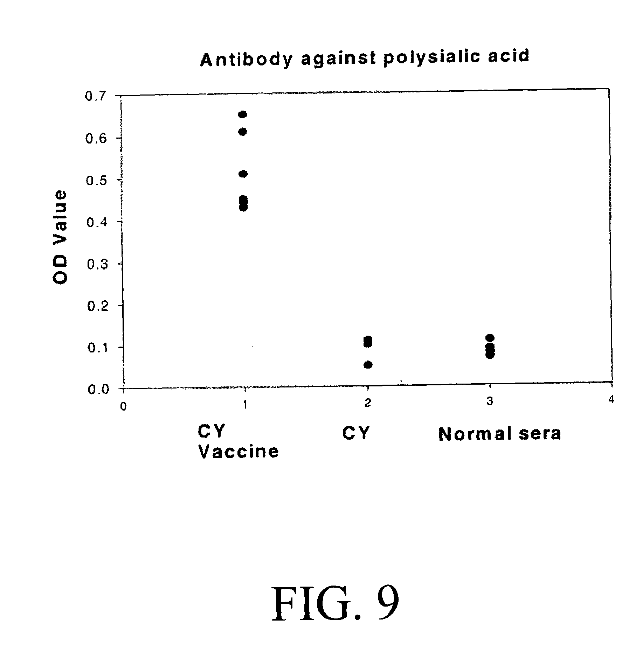

**Figure 9** illustrates that sera from the group treated by vaccine and cyclophosphamide also bind to polysialic acid. Sera from the group treated with cyclophosphamide alone and sera from the normal mice showed no binding with polysialic acid.

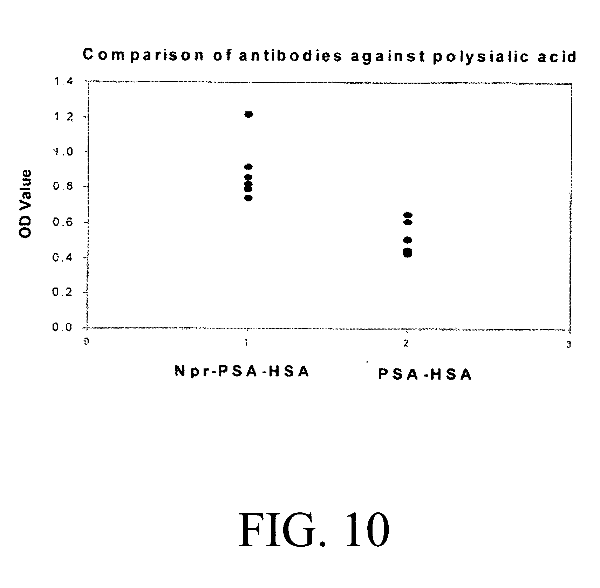

**Figure 10** provides a comparison of antibodies from the group treated by vaccine and cyclophophaminde against both NPr- and NAc- polysialic acids.

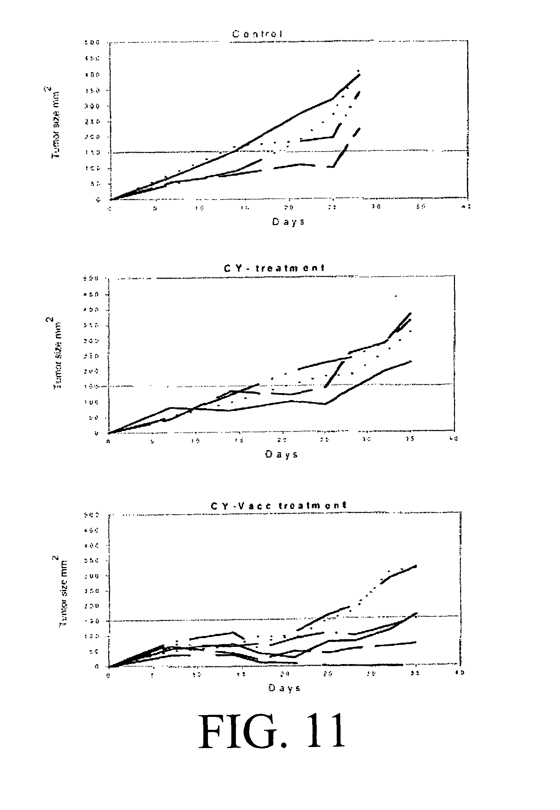

**Figure 11** illustrates the effect of vaccine on the growth of tumor *in vivo. 2* x 10⁶ RMA-S cells were injected for the development of tumors in mice. Mice were treated with cyclophosphamide (4 mg/mouse) at day 7 after inoculation of tumor cells (day 0). Whole cell vaccine (1-2 x 10⁶ /mouse) was administrated at day 8. The growth of the tumor was reduced by the whole cell vaccine, and was reduced further by the combination of whole cell vaccine with cyclophosphamide treatment.

**Table 3** provides data relating to determination of metastasis from the spleen of an individual mouse. The metastasis was determined by limiting dilution of spleen cells .

**Table 3**

| Metastasis from Spleen of Individual Mouse by Limiting Dilution | | | | | | | | | | |
|---|---|---|---|---|---|---|---|---|---|---|
| Treatment | Vaccine Group | | | | | | Control Group | | | |
| | RMA-S tumor CY (day 7*) Autologous Vaccine (day 8*) | | | | | | RMA-S tumor CY (day 7*) | | | |
| | - | - | - | - | - | - | + | - | + | + |
| Dilution of spleen cell suspension | 1:5 | 1:5 | 1:5 | 1:5 | 1:5 | 1:5 | 1:160 | 1:5 | 1:2560 | 1:60 |
| Percentage of spleen metastasis | 0 % | | | | | | 75 % | | | |

| | | | | | | | | | | |
|---|---|---|---|---|---|---|---|---|---|---|
| * Days indicated are following tumor graft at day 0. | | | | | | | | | | |

One fifth of cell suspension of spleen was used to initiate a serial 2-fold dilution. Cells were cultured over 20 days and tumor cells from the spleen were examined under the microscope. The negative score was the cell culture that had no tumor cells in comparison of the positive score with tumor cells in cell culture.

**Table 4** provides data summarizing two individual experiments for the detection of metastasis in mouse spleens. Metastasis was dramatically reduced when the vaccine was used in combination with the immunosupressive drug (CY).

**Table 4**

| Metastasis of Spleen (day 35) | | |
|---|---|---|
| Group | Vaccine + CY | CY |
| Percentage | 1/11 (9%) | (78%) |

The above-described embodiments of the present invention are intended to be examples only. Alterations, modifications and variations may be effected to the particular embodiments by those of skill in the art without departing from the scope of the invention, which is defined solely by the claims appended hereto.

### REFERENCES

1. O.T. Keppler, R. Horstkorte, M., Pawlita, C. Schmidt and W. Reutter (2001) Biochemical engineering of the N-acyl side chain of sialic acid: biological implications. Glycobiology 11, 11R-18R.
2. H. Kayser, R. Zeitler, C. Kannicht, D. Grunow, R. Nock, and N. Reutter (1992) Biosynthesis of nonphysiological sialic acid in different rat organs using N-propanoyl-D-hexosamines as precursors. J. Biol. Chem. 267, 16934-16938.
3. O.T. Keppler, P. Stehling, M. Herman, H. Kayser, D. Grunow, W. Reutter and M. Pawlita (1995) Biosynthetic modulation of sialic acid-dependent virus receptor interactions of two primate polyoma viruses. J. Biol. Chem. 270, 1308-1314.
4. U. Schumacher, D. Mukhtar, P. Stehling, W. Reutter (1996) Is the lectin binding pattern of human breast and colon cancer cells influenced by modulators of sialic acid metabolism?, Histochem. Cell Biol. 106, 599-604.
5. M. Herman, C.W. von der Leith, P. Stehling, W. Reutter and M. Pawlita (1997) Consequences of subtle sialic acid modification on the murine polyoma receptor. J. Virol. 71, 5922-5931.
6. L.K. Mahal, K.J. Yarema and C.R. Bertozzi (1997) Engineering chemical reactivity on cell surfaces through oligosaccharide biosynthesis. Science 276, 11256-1128.
7. K.J. Yarema, L.K. Mahal, R.E. Bruehl, E.C. Rodriguez and C.R. Bertozzi (1998) Metabolic delivery of ketone groups to sialic acid residues. Application to cell surface glycoform engineering. J. Biol. Chem. 275, 32832-32836.
8. B.E. Collins, T.J. Fralich, S. Itonori, Y. Ichikawa, R.L. Schnaar (2000) Conversion of cellular sialic acid expression from N-acetyl- to N-glycolylneuraminic acid using a synthetic precursor, N-glycolylmannosamine pentaacetate: inhibition of myelin-associated glycoprotein binding to neural cells. Glycobiology 10, 11-20.
9. T. Liu, Z. Guo, S. Sad and H.J. Jennings (2000) Biochemical engineering of surface a2-8 polysialic acid for immunotargeting tumor cells. J. Biol. Chem. 275, 32832-32836.
10. G.A. Lemieux and C.R. Bertozzi (2001) Modulating cell surface immunoreactivity by metabolic induction of unnatural carbohydrate antigens. Chem. Biol. 8, 265-275.
11. S.F. Slovin and H.J. Schen (1999) Peptide and carbohydrate vaccines in relapsed prostrate cancer: Immunogenicity of synthetic vaccines in man-clinical trials at Memorial Sloan-Kettering Cancer Center. Semin. Oncol. 26, 448-454.
12. K. Kärre, H.G. Ljunggren, G. Piontek, and R. Kiessling (1986) Selective rejection of H-2-deficient lymphoma variants suggests alternative immune defense strategy. Nature 319, 675-678.
13. R.A. Pon, M. Lussier, Q.L. Yang and H.J. Jennings (1997) N-propionylated group B meningococcal polysaccharide mimics a unique bactericidal capsular epitope in group B Neisseria meningitidis. J. Exp. Med. 185, 1929-1938.
14. M. Frosch, I. Gorgen, G.T. Boulnors and D. Bitter-Suermann (1985) NZB mouse system for production of monoclonal antibodies to weak bacterial antigens: isolation of an IgG antibody to the polysaccharide capsules of Escherichia coli K1 and group B meningococci. Proc. Natl. Acad Sci. U.S.A. 82, 1194-1198.
15. F.A. Troy (1992) Polysialylation: from bacteria to brains. Glycobiology 2,5-23.
16. J. Roth, C. Zuber, P. Komminoth, E.P. Scheidegger, M.J. Warhol, D. Bitter-Suermann and P.U. Heitz (1993) in Polysialic Acid (J. Roth, U. Rutishauser and F.A. Troy, eds.), pp. 335-348, Birkhauser Verlag, Basel, Switzerland.
17. C.M. Martersteck, N.L. Kedersha, D.A. Drapp, T.G. Tsui and K.J. Colley (1996) Unique a2-8-polysialylated glycoproteins in breast cancer and leukemic cells. Glycobiology 6, 289-301.
18. E.P. Scheidegger, P.M. Lackie, J. Papay and J. Roth (1994) In vitro and in vivo growth of clonal sublines of human small cell living carcinoma is modulated by polysialic acid of the neural cell adhesion molecule. Lab Invest. 70, 95-105.
19. P. Moingeon (2001) Cancer vaccines. Vaccine 19, 1305-1326.
20. D. Berd (2001) Autologous, hapten modified vaccine as a treatment for human cancers. Vaccine 19, 2565-2570.

## Claims

1. A killed immunogenic mammalian cancer cell having a cell surface marker incorporating a modified sialic acid unit capable of initiating an immune response in a mammalian system containing them which immune response is sufficiently strong to effectively combat proliferation of a live cancer cell.

2. The killed immunogenic mammalian cancer cell according to claim 1 wherein the modified sialic acid unit comprises an N-propionyl group, which may optionally be derived from N-propionyl mannosamine.

3. The killed immunogenic mammalian cancer cell of any one of claims 1 or 2, wherein said cell is either autologous to said mammalian system or allogeneic to said mammalian system.

4. An anti-cancer treatment comprising a pharmaceutically effective amount of a killed immunogenic mammalian cancer cell having a cell surface marker incorporating a modified sialic acid unit capable of initiating an immune response in a mammalian system containing them which immune response is sufficiently strong to effectively combat proliferation of a live cancer cell, in combination with a pharmaceutically acceptable carrier.

5. The anti-cancer treatment according to claim 4 wherein the modified sialic acid unit comprises an N-propionyl group, optionally derived from N-propionyl mannosamine.

6. An anti-cancer treatment comprising co-administration of a cell according to any one of claims 1 to 3 with N-propionyl mannosamine.

7. An anti-cancer treatment comprising co-administration of a cell according to any one of claims 1 to 3 with an anti-cancer drug selected from the group consisting of cyclophosphamide, melphalan, adriamycin, decarbazine, armustine, cisplatin, tamoxifen, bleomycine, vincristine and lomustine, and preferably wherein the anti-cancer drug comprises cyclophosphamide.

8. Use of a killed immunogenic mammalian cancer cell according to any one of claims 1 to 3 for preparation of an anti-cancer medicament.

9. An anti-cancer treatment comprising a pharmaceutically effective amount of a killed immunogenic mammalian cancer cell having a modified sialic acid unit on the surface thereof, said modified sialic acid unit comprising a N-propionyl polysialic acid, said cell being formed by, prior to killing the cell, incubating the cell with N-propionyl mannosamine, and subsequently killing the cell and combining the cell with a pharmaceutically acceptable carrier.

10. The anti-cancer treatment according to claim 9, wherein the cell is killed by heat treatment.
